# EUROPEAN PATENT SPECIFICATION

(11) **EP 2 840 088 B1**
(45) Date of publication and mention of the grant of the patent: **01.02.2017**
(21) Application number: 12874920.7
(22) Date of filing: 13.12.2012
(51) Int. Cl.: C07H 17/07

(54) **METHOD FOR PREPARING 5,6,4'-TRIHYDROXYFLAVONE-7-0-D-GLUCURONIC ACID**
VERFAHREN ZUR HERSTELLUNG VON 5,6,4 -TRIHYDROXYFLAVON-7-0-D-GLUCURONSÄURE
PROCÉDÉ DE PRÉPARATION D'ACIDE 5,6,4'-TRIHYDROXYFLAVONE-7-0-D-GLUCURONIQUE

(30) Priority: 18.04.2012 CN 201210114894
(43) Date of publication of application: 25.02.2015
(73) Proprietor: KPC Pharmaceuticals, Inc., Kunming, Yunnan 650106 (CN)
(72) Inventor: LI, Penghui, Kunming Yunnan 650106 (CN); ZHANG, Wei, Kunming Yunnan 650106 (CN); YANG, Zhaoxiang, Kunming Yunnan 650106 (CN); ZHANG, Xiongbo, Kunming Yunnan 650106 (CN); WANG, Jun, Kunming Yunnan 650106 (CN); ZHU, Haibei, Kunming Yunnan 650106 (CN); CHEN, Jinxin, Kunming Yunnan 650106 (CN); BAI, Yiyang, Kunming Yunnan 650106 (CN)
(74) Representative: Bösl, Raphael Konrad
(86) International application number: PCT/CN2012/086543
(87) International publication number: WO 2013/155849

(56) References cited:
- WO-A2-2008/100977
- CN-A- 1 840 537
- CN-A- 102 144 995
- CN-A- 102 146 067
- CN-A- 102 321 134
- CUI JIAN-MEI ET AL.: "Total synthesis of scutellarin-7- O -Glucuronide", JOURNAL OF ASIAN NATURAL PRODUCTS RESEARCH, vol. 7, no. 4, August 2005 (2005-08), pages 655-660, XP055183994,
- REN, XUHONG ET AL.: 'Graphical synthetic routes of Scutellarin-7-O-glucuronide' CHINA MEDICAL HERALD. vol. 5, no. 3, January 2008, pages 23 - 24, XP008175256

## Description

### FIELD OF THE INVENTION

The present invention belongs to the field of drug synthesis, and particularly relates to a method for preparing 5,6,4'-trihydroxyflavone-7-O-D-glucuronic acid.

### BACKGROUND OF THE INVENTION

5,6,4'-trihydroxyflavone-7-O-D-glucuronic acid, also called scutellarin or breviscapine, is the main active ingredient in flavonoids mixtures extracted from the herba of *Erigeron breviscapus* (also called herba erigerontis) of *Erigeron, Compositae,* which has a molecular formula of C₂₁H₁₈O₁₂ and a molecular weight of 462.37, with a structural formula as shown in Formula I:

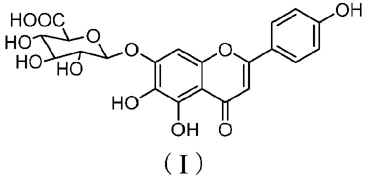

Clinical studies have indicated that 5,6,4'-trihydroxyflavone-7-O-D-glucuronic acid has good therapeutic effect on hypertension, hematencephalon, cerebral thrombosis, cerebral embolism polyneuritis, chronic arachnoiditis and sequela thereof, and also has some therapeutic effect on rheumatism and coronary heart disease. In addition, extensive studies have demonstrated that 5,6,4'-trihydroxyflavone-7-O-D-glucuronic acid has anti-inflammatory, anti-oxidant and protein kinase inhibition effects, but also has therapeutic effect on diabetes, nephrosis, rheumatism and rheumatoid and other diseases. Therefore, 5,6,4'-trihydroxyflavone-7-O-D-glucuronic acid currently has been widely used in clinic for treating paralysis caused by cerebrovascular accidents such as cerebral thrombosis, cerebral infarction, polyneuritis, chronic arachnoiditis or the like, as well as for treating coronary heart disease, angina, gouty arthritis and other conditions.

Recently, 5,6,4'-trihydroxyflavone-7-O-D-glucuronic acid is mostly obtained by extracting and isolating from plants, which has a low extraction efficiency and high extraction cost, failing to meet the requirements for current studies and production, thus extremely limiting its development and application in the biomedical field. Therefore, it is of great significance to prepare 5,6,4'-trihydroxyflavone-7-O-D- glucuronic acid by using chemical synthesis.

There are many patents and literatures relating to preparation of 5,6,4'-trihydroxyflavone-7-O-D-glucuronic acid by using chemical synthesis method, such as Chinese patents with publication numbers of CN101538298A, CN101538299A and CN101941999A respectively, as well as a literature "Total synthesis of scutellarin-7-O-Glucuronide" (CUI Jianmei et al., Journal of Asian Natural Products Research , 2005,7(4): 655-660). However, the current preparation method is too complicated in operation, has an unduly long reaction route and a lower yield, and more importantly, during selective reduction and glycosylation, it also uses silver compounds and palladium carbon, etc. added in an equivalent amount, which are expensive, thus significantly increasing the production cost. Although it is theoretically applicable to use a mercuric salt to replace the silver salt as the catalyst for catalyzing glycosidation, and expectedly there will be a better result, massive use of a mercuric salt will possibly lead to pressure for environmental protection and increase costs due to high toxicity of the mercuric salt, and at the same time, may cause huge obstacles to the quality inspection on raw drug materials, which is detrimental to the industrial production.

### SUMMARY OF THE INVENTION

In view of these, an objective of the present invention is aiming at the defects that the synthetic methods of 5,6,4'-trihydroxyflavone-7-O-D-glucuronic acid in the prior art have a long synthetic route and low yield, use reagents which are expensive and with large quantities, and have complicated process operations and rigorous reaction conditions, to provide a method for preparing 5,6,4'-trihydroxyflavone-7-O-D-glucuronic acid which has a short synthetic route, high yield and low cost.

In order to achieve the objective of the present invention, the following technical solution is adopted in the present invention:
Step 1: an acylation reaction of 5,6,7,4'-tetrahydroxyflavone with an acylation reagent, to generate a compound shown in formula II, wherein the acylation reagent is pivaloyl chloride, benzoyl chloride, acetic anhydride, acetic acid or acetyl chloride;
Step 2: an glycosylation reaction of an acyloxy group on position 7 of the compound shown in formula II with methyl α-bromotriacetoxy glucuronate in the presence of a catalyst, to generate a compound shown in formula III, the catalyst being one or more of potassium carbonate, potassium iodide or potassium bromide;
Step 3: a hydrolysis reaction of the compound shown in formula III with the catalysis of an inorganic acid, to generate a compound shown in formula IV; and
Step 4: a hydrolysis reaction of the compound shown in formula IV with the catalysis of an inorganic base under oxygen-free conditions, followed by a neutralization reaction by adding an inorganic acid to adjust pH of the reaction solution to 2~3, thus obtaining the 5,6,4'-trihydroxyflavone-7-O-D-glucuronic acid; 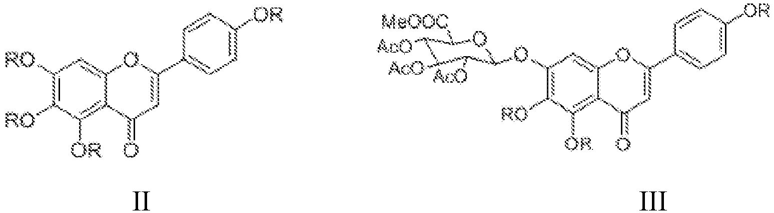 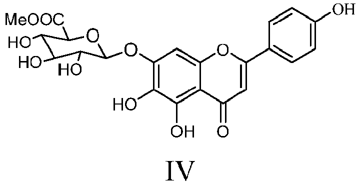 wherein R is selected from a trimethylacetyl, benzoyl or acetyl group; Me is methyl and Ac is acetyl.

The preparing method of the present invention uses 5,6,7,4'-tetrahydroxyflavone as a raw material, to obtain 5,6,4'-trihydroxyflavone-7-O-D-glucuronic acid through a four-step reaction including an acylation reaction, a glycosylation reaction, acid hydrolysis and then base hydrolysis followed by acid neutralization.

Firstly, for the acylation reaction of 5,6,7,4'-tetrahydroxyflavone with an acylation reagent in step 1 of the preparing method of the present invention, hydrogen atoms of hydroxyl groups in 5,6,7,4'-tetrahydroxyflavone are replaced with acyl groups, thus generating a compound shown in formula II, and the reaction equation is as follows:

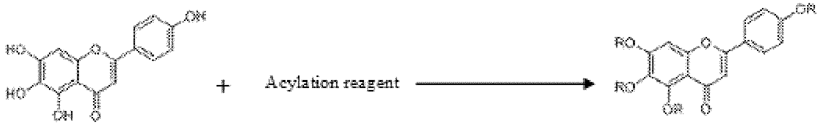

The acylation reagent of the present invention is pivaloyl chloride, benzoyl chloride, acetic anhydride, acetic acid or acetyl chloride. Therefore, the hydrogen atoms of hydroxyl groups in the 5,6,7,4'-tetrahydroxyflavone are replaced with trimethylacetyl, benzoyl or acetyl groups, to generate 5,6,7,4'-tetrapivaloyloxyflavone, 5,6,7,4'-tetrabenzoyloxyflavone and 5,6,7,4'-tetraacetoxyflavone, respectively.

Preferably, the molar ratio of the acylation reagent to the 5,6,7,4'-tetrahydroxyflavone in step 1 is 5:1~50:1, preferably 10:1~30:1, and more preferably 20:1.

The catalyst refers to a material capable of increasing chemical reaction rate while having no permanent change in its structure *per se.* The acylation reaction in step 1 of the present invention is preferably carried out under the action of the catalyst to reduce the reaction time and improve the reaction yield. Preferably, the catalyst of the acylation reaction in step 1 is one or more of pyridine, dichloromethane, toluene, tetrahydrofuran, 1,4-dioxane or ethyl acetate.

The temperature of the acylation reaction in step 1 of the present invention is generally 80-300 °C, and is 100-200 °C in certain embodiments, is 130 °C in some embodiments, is 180 °C in some embodiments, and is 140 °C in some embodiments.

The time for the acylation reaction of the present invention is generally 3-20 h, is 4~10 h in certain embodiments, is 6~8 h in some embodiments, is 9 h in some embodiments, and is 4~5 h in some embodiments.

For step 2 of the preparing method of the present invention, a glycosylation reaction of the compound shown in formula II with methyl α-bromotriacetoxy glucuronate takes place in the presence of a catalyst, to generate a compound shown in formula III, and the catalyst is one or more of potassium iodide and potassium bromide, and the reaction equation is as follows:

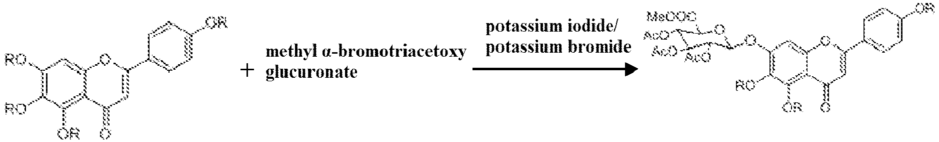

Preferably, the molar ratio of the catalyst to the compound shown in formula II in step 2 of the preparing method of the present invention is 0.1:1-20:1, and more preferably 0.3:1~5:1. In some embodiments, the ratio is 3:1; in some embodiments, the ratio is 2.5:1; and in some embodiments, the ratio is 0.45:1.

The solvent is a liquid which can dissolve solid, liquid or gas solutes, thus forming a solution. The solvent cannot react with the solutes, and is inert, and usually has a relatively low boiling point and thus volatile, or may be removed by distillation, thus leaving the dissolved solutes. Preferably, the solvent for the glycosylation reaction in step 2 of the preparing method of the present invention is one or more of methanol, ethanol, acetone, butanone, dimethylformamide, dimethylacetamide, dimethyl sulfoxide, tetrahydrofuran and dichloromethane.

The temperature of the glycosylation reaction in step 2 of the present invention is generally 0~200 °C, and is room temperature in some embodiments.

The time of the glycosylation reaction of the present invention is generally 3-24 h, is 15~20 h in some embodiments and is 18 h in some embodiments.

In some embodiments, the glycosylation reaction in step 2 of the preparing method of the present invention activates the phenolic hydroxyl group on position 7 under basic conditions. Preferably, the base is potassium carbonate.

For step 3 of the preparing method of the present invention, a hydrolysis reaction of the compound III shown in formula III is performed under the catalysis of an inorganic acid, to generate a compound shown in formula IV, of which the reaction equation is as follows:

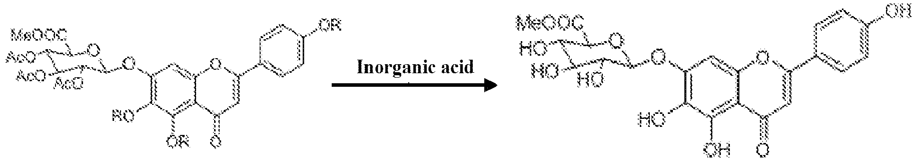

Preferably, the inorganic acid in step 3 is sulfuric acid or nitric acid.

The solvent for the hydrolysis reaction in step 3 of the preparing method of the present invention is preferably one or more of acetone, water, methanol, ethanol, dichloromethane, dimethyl sulfoxide, tetrahydrofuran or dioxane.

Preferably, the volume ratio of the inorganic acid to the solvent for the hydrolysis reaction in step 3 is 0.005:1-0.1:1, is 0.01:1~0.02:1 in certain embodiments, is 0.015:1 in some embodiments, and is 0.017:1 in some embodiments.

The temperature of the hydrolysis reaction in step 3 of the present invention is generally 30 °C~250 °C, and it is a heating reflux reaction in certain embodiments.

The time for the hydrolysis reaction of the present invention is generally 5-20 h, and is 8 h in some embodiments.

For step 4 of the preparing method of the present invention, the hydrolysis reaction of the compound shown in IV takes place with the catalysis of an inorganic base under oxygen-free conditions, to generate 5,6,4'-trihydroxyflavone-7-O-D-glucuronic acid, and the reaction equation is as follows: 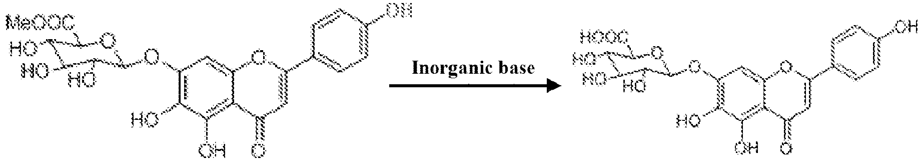 wherein the oxygen-free conditions may be the conditions under the protection of nitrogen gas, argon gas, helium gas or a mixed gas thereof.

Preferably, the inorganic base in step 4 is one or more of sodium hydroxide, lithium hydroxide, potassium hydroxide, potassium carbonate or sodium carbonate.

Preferably, the molar ratio of the inorganic base to the compound shown in formula IV in step 4 is 0.1:1-50:1; in certain embodiments, the ratio is 1:1-10:1; in some embodiments, the ratio is 4:1; in some embodiments, the ratio is 2.5:1; and in some embodiments, the ratio is 5:1.

The solvent for the hydrolysis reaction in step 4 of the preparing method of the present invention is preferably one or more of acetone, water, methanol, ethanol, dichloromethane, dimethyl sulfoxide, tetrahydrofuran or dioxane.

The temperature of the hydrolysis reaction in step 4 of the present invention is generally -10 °C~40 °C, and in certain embodiments the temperature is 0 °C.

The time for the hydrolysis reaction of the present invention is generally 1~10 h; in certain embodiments, the time is 2-4 h; in some embodiments the time is 2 h; and in some embodiments the time is 3 h.

For step 4 of the method of the present invention, inorganic acid is added after the hydrolysis reaction to neutralize the inorganic base in the hydrolysis reaction solution. Preferably, the inorganic acid is one or more of hydrochloric acid, sulfuric acid, acetic acid, formic acid, nitric acid or phosphoric acid. The temperature of the neutralization reaction is similar with that of the hydrolysis reaction, preferably -10 °C~40 °C, and in some embodiments, the temperature is 0 °C.

The method for preparing 5,6,4'-trihydroxyflavone-7-O-D-glucuronic acid of the present invention uses 5,6,7,4'-tetrahydroxyflavone as a raw material and adopts a new synthetic route to obtain high-purity of 5,6,4'-trihydroxyflavone-7-O-D-glucuronic acid through a four-step reaction including an acylation reaction, a glycosylation reaction, acid hydrolysis and base hydrolysis followed by neutralization. The preparing method of the present invention has a short synthetic route, low cost, and high reaction yield, the product is easy for purification, and the method is applicable to industrial production of 5,6,4'-trihydroxyflavone-7-O-D-glucuronic acid. Compared with the prior art, the preparing method of the present invention has the following advantages:
1. avoiding the conventional reaction mode of traditional process, omitting the protection and selective deprotection steps for the phenolic hydroxyl groups in parent flavonoids, shorting the reaction route, significantly improving the reaction rate and yield, and reducing the production cost;
2. using two-step hydrolysis of the compound shown in formula III to obtain 5,6,4'-trihydroxyflavone-7-O-D-glucuronic acid, which makes the reaction more moderate and significantly improves the reaction yield;
3. optimizing each reaction step, shorting the reaction time, improving the reaction yield and reducing the cost;
4. having high purity of products, which reaches up to 98% or more as detected by HPLC for 5,6,4'-trihydroxyflavone-7-O-D-glucuronic acid; and
5. the raw materials for the reaction being abundant in source and cheap in price, and having low toxicity and environmental protection for each reaction step.

### DESCRIPTION OF THE DRAWINGS

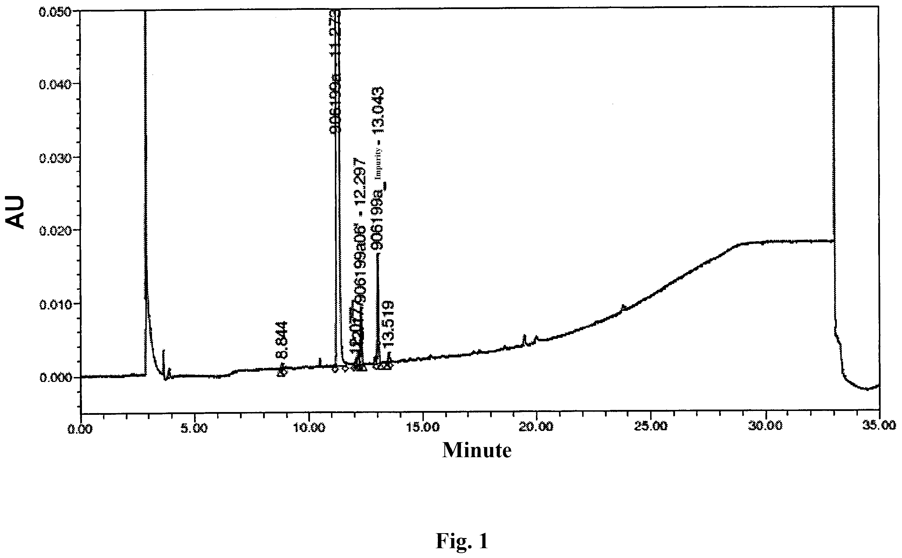

Figure 1 shows a chromatogram detected by HPLC for the 5,6,4'-trihydroxyflavone-7-O-D-glucuronic acid prepared in Example 1.

### DETAILED EMBODIMENTS

The Example of the present invention discloses a method for preparing 5,6,4'-trihydroxyflavone-7-O-D-glucuronic acid. Those skilled in the art may use the content herein for reference and achieve the present invention through appropriate improvements on process parameters. It should be particularly noted that all the similar substitutions and modifications are obvious for those skilled in the art, which are deemed to be included in the present invention. The method of the present invention has been described by preferred examples, and those skilled in the art can obviously make modifications or appropriate changes or combinations on the method described herein without departing from the content, spirit and scope of the present invention, to achieve and apply the technique of the present invention.

For further understanding the present invention, it is illustrated in details in conjunction with Examples as follows. Raw materials and reagents used in the examples are all commercially available, and the calculation formula for the yield of the synthesized compound is: actual generation amount of reaction/theoretical generation amount of reaction × 100%. The amount of the reactant in each step of the examples has no effect on the use of other steps, and if the amount of the reaction product cannot satisfy the use for other steps, it may be prepared for many times to satisfy the amount required by other steps. The compound with a structural formula as shown in formula IV is referred to as compound IV for short.

### Example 1:

### 1. Preparation of 5,6,7,4'-tetrapivaloyloxyflavone

10 g (70 mmol) of 5,6,7,4'-tetrahydroxyflavone was dissolved in 60 mL of pyridine, and 86 mL of pivaloyl chloride (d=0.976 g/mL, 1.4 mol) was slowly added dropwise at room temperature, then heated to 130 °C with a reflux reaction for 6~8 hours. After the reaction was completed as detected by TLC, the reaction solution was cooled to about 70 °C, and 100 mL of ethyl acetate was added , with large amount of white solid separated out gradually. It was stirred continuously for 1 hour and cooled to room temperature, then left standing overnight in a freezer. It was filtered to obtain 5,6,7,4'-tetrapivaloyloxyflavone as an off-white solid. The resulting solid was dried and then recrystallized with ethanol to obtain 18 g of pure 5,6,7,4'-tetrapivaloyloxyflavone, which was a white product with a chromatographic purity above 98.00% and a yield of 84%.

1H-NMR (400MHz, CDCl3), δ (ppm): 7.75 (2H, d, J=8.6 Hz), 7.13 (1H, s), 7.12 (2H, d, J= 8.6Hz), 6.48 (1H, s), 1.59 (9H, s), 1.54(9H, s), 1.52 (9H, s), 1.51 (9H, s).

### 2. Preparation of methyl 5,6,4'-tripivaloyloxyftavone-7-O-D-triacetyloxy glucuronate

200 mL of N,N-dimethylformamide was added to 14 g (22 mmol) of 5,6,7,4'-tetrapivaloyloxyflavone, and 7 g (50 mmol) of potassium carbonate, 880 mg (5.3 mmol) of potassium iodide and 26.2 g (66 mmol) of methyl α-bromotriacetoxy glucuronate were added sequentially under stirring at room temperature. The reaction solution was stirred for about 18 hours at room temperature, and the reaction was completed as detected by HPLC. The reaction solution was filtered, and the filtrate was evaporated to dryness under reduced pressure, to obtain a crude product of methyl 5,6,4'-tripivaloyloxyflavone-7-O-D-triacetyloxy glucuronate. It was rinsed with 150 mL of ethanol followed by filtering by suction to dryness; then recrystallized with ethanol after dried to obtain 12.2 g of pure methyl 5,6,4'-tripivaloyloxyflavone-7-O-D-triacetyloxy glucuronate, with a chromatographic purity of about 98.0% and a yield of 65%.

1H-NMR (400MHz, DMSO), δ (ppm): 8.10 (2H, d, J=8.6 Hz), 7.53 (1H, s), 7.37 (2H, d, J= 8.6Hz), 6.56 (1H, s), 6.45 (1H, d, J= 7.8Hz), 5.88 (1H, d, J= 7.8Hz), 5.47 (1H, m), 5.20 (1H, m), 5.10 (1H, m), 4.84 (1H, d, J=10Hz), 3.85 (3H, s), 2.51 (3H, s), 2.47 (3H, s), 2.33 (3H, s), 1.75(9H, s), 1.73 (9H, s), 1.72 (9H, s).

### 3. Preparation of Compound IV

1 L of anhydrous methanol was added to 40 g (46 mmol) of methyl 5,6,4'-tripivaloyloxyflavone-7-O-D-triacetyloxy glucuronate, and 15 mL of concentrated sulfuric acid was added dropwise under stirring. The reaction solution was heated with reflux for 8 hours, and the reaction was completed as detected by TLC. The reaction solution was cooled to room temperature, left standing for 5 hours and then filtered by suction. The filter cake was washed with 300 mL of anhydrous methanol, dried and then recrystallized to obtain 20 g of compound IV, with a chromatographic purity above 98.0% and a yield of 90%.

1H-NMR (400MHz, DMSO), δ (ppm): 7.89~7.91 (2H, d, J = 7.2 Hz ), 6.97 (1H, s), 6.90-6.92(2H, d, J = 7.6 Hz ), 6.79 (1H, s), 5.24~5.25 (1H, m), 4.16~4.19 (2H, m), 3.65 (3H, s), 3.58 (1H, s), 3.32~3.39 (3H, m), 3.14 (1H, s).

### 4. Preparation of 5,6,4'-trihydroxyflavone-7-O-D-glucuronic acid

A mixed solution of 300 mL water and 300 mL acetone was added to 20 g (42 mmol) of compound IV, and 70 mL of 10% aqueous sodium hydroxide was added dropwise under stirring at 0 °C. The reaction was stirred continuously under protection of nitrogen gas at 0 °C for about two hours, which was completed as detected by HPLC. 10% aqueous hydrochloric acid was added dropwise under stirring, to adjust pH to 2.0~3.0, and stirred continuously for 1 hour followed by gradually raising to room temperature, with large amount of yellow solid 5,6,4'-trihydroxyflavone-7-O-D-glucuronic acid gradually separated out. The reaction mixture was left standing overnight at room temperature, filtered by suction, and the filter cake was washed with suitable amount of water and acetone, dried and then recrystallized with ethanol to obtain 17.3 g of pure 5,6,4'-trihydroxyflavone-7-O-D-glucuronic acid, with a yield of 89%. The purity was 98.74% detected by HPLC, the chromatogram detected by HPLC was shown in Figure 1, and the statistical result was shown in table 1.

1H-NMR (400MHz, DMSO), δ (ppm): 10.41 (1H, s), 8.63 (1H, s), 7.94 (2H, d, J= 8.8Hz), 6.99 (1H, s), 6.94 (2H, d, J= 8.8Hz), 6.86 (1H, s), 5.51-5.31 (3H, br), 5.28 (1H, d, J = 7.5 Hz), 4.11 (2H, d, J=9.6Hz), 3.43 (4H, m).

**Table 1 Chromatographic results detected by HPLC**

| Peak No. | Retention time (min) | Area | Area percentage (%) |
|---|---|---|---|
| 1 | 8.844 | 4283 | 0.05 |
| 2 | 11.273 | 8602276 | 98.74 |
| 3 | 12.077 | 4465 | 0.05 |
| 4 | 12.177 | 6987 | 0.08 |
| 5 | 12.297 | 31942 | 0.37 |
| 6 | 13.043 | 56188 | 0.64 |
| 7 | 13.519 | 5752 | 0.07 |

### Example 2:

### 1. Preparation of 5,6,7,4'-tetrabenzoyloxyflavone

10 g (35 mmol) of 5,6,7,4'-tetrahydroxyflavone was dissolved in 70 mL of pyridine, and 81 mL of benzoyl chloride (d=1.212, 700 mmol) was slowly added under stirring at room temperature, then heated to 180 °C with a reflux reaction for about 9 hours. After the reaction was completed as detected by TLC, the reaction solution was cooled to about 70 °C and added 90 mL of ethyl acetate, with large amount of white solid gradually separated out. It was stirred continuously for 1 hour and cooled to room temperature, then left standing overnight in a freezer. It was filtered to obtain 5,6,7,4'-tetrabenzoyloxyflavone as an off-white solid. The resulting solid was dried and then recrystallized with ethanol to obtain 21.4 g of pure 5,6,7,4'-tetrabenzoyloxyflavone, which was an off-white product with a chromatographic purity above 98.0% and a yield of 87%.

1H-NMR (400MHz, DMSO), δ (ppm): 8.56 (2H, d, J=8.9Hz), 8.55 (2H, d, J=8.9Hz ), 8.54 (2H, d, J=8.9Hz ), 8.52 (2H, d, J=8.9Hz ), 8.10 (2H, d, J=8.6 Hz), 8.06(1H, m), 8.09-8.01 (4H, m), 7.95 (2H, d, J=8.7Hz ), 7.94 (2H, d, J=8.7Hz ), 7.92 (2H, d, J=8.7Hz ), 7.91 (2H, d, J=8.6Hz), 7.67 (2H, d, J= 8.6Hz), 7.43 (1H, s), 6.98 (1H, s).

### 2. Preparation of methyl 5,6,4'-tribenzoyloxyflavone-7-O-D-triacetyloxy glucuronate

250 mL of acetone was added to 16 g (22 mmol) of 5,6,7,4'-tetrabenzoyloxyflavone, and 7 g (50 mmol) of potassium carbonate, 880 mg (5.3 mmol) of potassium iodide and 26.2 g (66 mmol) of methyl α-bromotriacetoxy glucuronate were added sequentially under stirring at room temperature. The reaction solution was stirred for about 18 hours at room temperature, and the reaction was completed as detected by HPLC. The reaction solution was filtered, and the filtrate was distilled to dryness under a reduced pressure, to obtain a crude product of methyl 5,6,4'-tribenzoyloxyflavone-7-O-D-triacetyloxy glucuronate. It was recrystallized with ethanol to obtain 12 g of pure methyl 5,6,4'-tribenzoyloxyflavone-7-O-D-triacetyloxy glucuronate, with a yield of 60% and a chromatographic purity above 98.0% detected by HPLC.

1H-NMR (400MHz, DMSO), δ (ppm) 8.76 (2H, d, J=8.9Hz), 8.67(2H, d, J=8.9Hz ), 8.58 (2H, d, J=8.9Hz ), 8.52 (2H, d, J=8.9Hz), 8.31 (2H, d, J=8.6 Hz), 8.16(1H, m), 8.11-8.04 (4H, m), 7.96 (2H, d, J=8.7Hz ), 7.94 (2H, d, J=8.7Hz ), 7.91 (2H, d, J=8.7Hz ), 7.87 (2H, d, J=8.6Hz), 7.64 (2H, d, J= 8.6Hz), 6.99 (1H, s), 6.97 (1H, s), 6.47 (1H, d, J= 7.8Hz), 5.95 (1H, d, J= 7.8Hz), 5.56 (1H, m), 5.29 (1H, m), 5.16 (1H, m), 4.91 (1H, d, J=10Hz), 3.94 (3H, s), 2.62 (3H, s), 2.56 (3H, s), 2.42 (3H, s).

### 3. Preparation of Compound IV

1 L of anhydrous methanol was added to 42 g (46 mmol) of methyl 5,6,4'-tribenzoyloxyflavone-7-0-D-triacetyloxy glucuronate, and 17 mL of concentrated nitric acid was added dropwise under stirring. The reaction solution was heated with reflux for about 8 hours, and the reaction has been completed as detected by TLC. The reaction solution was cooled to room temperature and left standing for 5 hours and then filtered. The filter cake was washed with 300 mL of anhydrous methanol, dried and then recrystallized to obtain 19 g of compound IV, with a yield of 88% and a chromatographic purity above 98.00% detected by HPLC.

1H-NMR (400MHz, DMSO), δ (ppm): 7.89~7.91 (2H, d, J = 7.2 Hz), 6.97 (1H, s), 6.90-6.92(2H, d, J = 7.6 Hz), 6.79 (1H, s), 5.24~5.25 (1H, m), 4.16~4.19 (2H, m), 3.65 (3H, s), 3.58 (1H, s), 3.32~3.39 (3H, m), 3.14 (1H, s) .

### 4. Preparation of 5,6,4'-trihydroxyflavone-7-O-D-glucuronic acid

A mixed solution of 300 mL water and 300 mL acetone was added to 20 g (42 mmol) of compound IV, and 60 mL of 10% aqueous solution of potassium hydroxide was added dropwise under stirring at 0 °C. The reaction was stirred continuously under protection of nitrogen gas at 0 °C for about two hours, which was completed as detected by HPLC. 10% aqueous solution of hydrochloric acid was added dropwise under stirring, to adjust pH to 2.0~3.0, and stirred continuously for 1 hour followed by gradually raising to room temperature, with large amount of yellow solid 5,6,4'-trihydroxyflavone-7-O-D-glucuronic acid separated out. The reaction mixture was left standing overnight at room temperature, filtered by suction, and the filter cake was washed with suitable amount of water and acetone, dried and recrystallized with ethanol to obtain 16.7 g of pure 5,6,4'-trihydroxyflavone-7-O-D-glucuronic acid, with a yield of 86%. The purity was above 98.00% detected by HPLC, and the detection result of NMR was identical to that of Example 1.

### Example 3:

### 1. Preparation of 5,6,7,4'-tetraacetoxyflavone

10 g (35 mmol) of 5,6,7,4'-tetrahydroxyflavone was dissolved in 50 mL of pyridine, and 30 mL of acetic anhydride (d=1.08, 400 mmol) was slowly added dropwise at room temperature, then heated to 140 °C with a reflux reaction for about 4~5 hours. After the reaction was completed as detected by TLC, the reaction solution was cooled to about 70 °C, and 90 mL of ethyl acetate was added, with large amount of white solid gradually separated out. It was stirred continuously for 1 hour and cooled to room temperature, then left standing overnight in a freezer. It was filtered to obtain 5,6,7,4'-tetraacetoxyflavone as an off-white solid. The resulting solid was dried and then recrystallized with ethanol to obtain 13.7 g of pure 5,6,7,4'-tetraacetoxyflavone which was off-white pure product, with a chromatographic purity above 98.00% and a yield of 86%.

1 H-NMR (400MHz, CDC13), δ (ppm): 7.64 (2H, d, J=8.7 Hz), 7.03 (1H, s), 7.02 (2H, d, J= 8.6Hz), 6.39 (1H, s), 2.21(3H, s), 2.14 (3H, s), 2.12 (3H, s), 2.11 (3 H, s).

### 2. Preparation of methyl 5,6,4'-triacetoxyflavone-7-O-D-triacetyloxy glucuronate

150 mL of N,N-dimethylformamide was added to 10 g (22 mmol) of 5,6,7,4'-tetraacetoxyflavone, and 6 g (4.4 mmol) of potassium carbonate, 913 mg (5.5 mmol) of potassium iodide and 26.2 g (66 mmol) of methyl α-bromotriacetoxy glucuronate were added sequentially under stirring at room temperature. The reaction solution was stirred for about 18 hours at room temperature, and the reaction was completed as detected by HPLC. After 20 mL of 10% hydrochloric acid was added dropwise to the reaction solution for acidification, 500 mL of water was further added with continuously stirring for 30 minutes, with large amount of solid separated out. It was left standing for about half an hour then filtered, and the filter cake was rinsed with 150 mL of ethanol followed by filtering by suction to dryness, to obtain a crude product of methyl 5,6,4'-triacetoxyflavone-7-O-D-triacetyloxy glucuronate. It was dried and then recrystallized with ethanol to obtain 11 g of pure methyl 5,6,4'-triacetoxyflavone-7-O-D-triacetyloxy glucuronate, with a yield of 69% and a chromatographic purity above 98% detected by HPLC.

1H-NMR (500MHz, DMSO), δ (ppm): 8.10 (2H, d, J=8.6 Hz), 7.53 (1H, s), 7.37 (2H, d, J= 8.6Hz), 6.88 (1H, s), 5.88 (1H, d, J= 7.8Hz), 5.47 (1H, m), 5.20 (1H, m), 5.10 (1H, m), 4.84 (1H, d, J=10Hz), 3.65 (3H, s), 2.33 (3H, s), 2.31 (3H, s), 2.27 (3H, s), 2.02 (3H, s), 2.01 (3H, s), 1.99(3H, s).

### 3. Preparation of Compound IV

1 L of anhydrous methanol was added to 40 g (46 mmol) of methyl 5,6,4'-triacetoxyflavone-7-O-D-triacetyloxy glucuronate, and 15 mL of concentrated sulfuric acid was added dropwise under stirring. The reaction solution was heated with reflux for about 8 hours, and the reaction has been completed as detected by TLC. The reaction solution was cooled to room temperature and left standing for 5 hours and then filtered by suction. The filter cake was washed with 300 mL of anhydrous methanol, dried and then recrystallized to obtain 20 g of compound IV, with a yield of 90% and a chromatographic purity above 98.00% detected by HPLC.

1H-NMR (400MHz, DMSO), δ (ppm): 7.89~7.91 (2H, d, J = 7.2 Hz), 6.97 (1 H, s), 6.90-6.92(2H, d, J = 7.6 Hz), 6.79 (1H, s), 5.24~5.25 (1H, m), 4.16~4.19 (2H, m), 3.65 (3H, s), 3.58 (1H, s), 3.32~3.39 (3H, m), 3.14 (1H, s).

### 4. Preparation of 5,6,4'-trihydroxyflavone-7-O-D-glucuronic acid

A mixed solvent of 100 mL acetone and 100 mL water was added to 7.3 g (10 mmol) of methyl 5,6,4'-triacetoxyflavone-7-O-D-triacetyloxy glucuronate, and 110 mL of 10% aqueous solution of lithium hydroxide was slowly added dropwise under the protection of nitrogen gas at -7 °C. The reaction was stirred for about four hours, which was completed as detected by TLC. 2 mol/L aqueous solution of hydrochloric acid was slowly added dropwise to the reaction solution at -7 °C, to adjust its pH to 2-3, and stirred continuously for 1 hour followed by gradually raising to room temperature, with large amount of yellow solid 5,6,4'-trihydroxyflavone-7-O-D-glucuronic acid gradually separated out from the reaction solution. It was left standing overnight at room temperature, filtered, and the filter cake was washed with suitable amount of water and acetone, dried and then recrystallized with ethanol to obtain 3.8 g of 5,6,4'-trihydroxyflavone-7-O-D-glucuronic acid, with a yield of 83%. The purity was above 98.00% detected by HPLC, and the detection result of NMR was identical to that of Example 1.

A mixed solution of 300 mL water and 300 mL acetone was added to 20 g (42 mmol) of compound IV, and 85 mL of 10% aqueous solution of lithium hydroxide was added dropwise under stirring at 0 °C under the protection of nitrogen. The reaction was stirred continuously at 0 °C for about three hours, which was completed as detected by HPLC. 10% aqueous solution of hydrochloric acid was added dropwise under stirring, to adjust pH to 2.0~3.0, and stirred continuously for 1 hour followed by gradually raising to room temperature, with large amount of yellow solid 5,6,4'-trihydroxyflavone-7-0-D-glucuronic acid separated out. The reaction mixture was left standing overnight at room temperature, filtered by suction, and the filter cake was washed with suitable amount of water and acetone, dried and recrystallized with ethanol to obtain 16.3 g of pure 5,6,4'-trihydroxyflavone-7-O-D-glucuronic acid, with a yield of 84%. The purity was above 98.00% detected by HPLC, and the detection result of NMR was identical to that of Example 1.

The illustration of the forgoing examples is only for helping to understand the method of the present invention and its core idea.

## Claims

1. A method for preparing 5,6,4'-trihydroxyflavone-7-O-D-glucuronic acid, **characterized in that** the method comprises:
step 1: an acylation reaction of 5,6,7,4'-tetrahydroxyflavone with an acylation reagent, to generate a compound shown in formula II, wherein the acylation reagent is pivaloyl chloride, benzoyl chloride, acetic anhydride, acetic acid or acetyl chloride;
step 2: an glycosylation reaction of an acyloxy group on position 7 of the compound shown in formula II with methyl α-bromotriacetoxy glucuronate in the presence of a catalyst, to generate a compound shown in formula III, the catalyst being one or more of potassium iodide and potassium bromide;
step 3: a hydrolysis reaction of the compound shown in formula III under the catalysis of an inorganic acid, to generate a compound shown in formula IV; and
step 4: a hydrolysis reaction of the compound shown in formula IV under the catalysis of an inorganic base under an oxygen-free condition, followed by a neutralization reaction by adding an inorganic acid to adjust pH of the reaction solution to 2~3, thus obtaining the 5,6,4'-trihydroxyflavone-7-O-D-glucuronic acid; 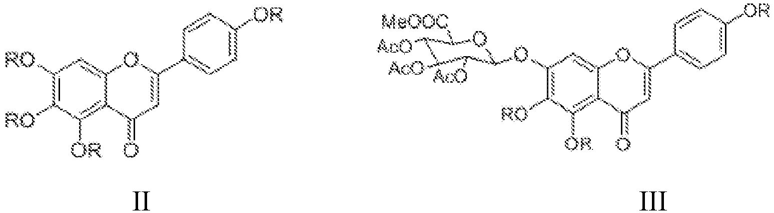 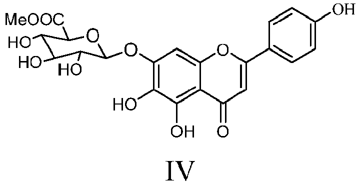 wherein R is selected from the group consisting of trimethylacetyl, benzoyl or acetyl; Me is methyl; and Ac is acetyl.

2. The method according to claim 1, **characterized in that** the molar ratio of the acylation reagent to the 5,6,7,4'-tetrahydroxyflavone in step 1 is 5:1~50:1.

3. The method according to claim 1, **characterized in that** the catalyst for the acylation reaction in step 1 is one or more of pyridine, dichloromethane, toluene, tetrahydrofuran, 1,4-dioxane and ethyl acetate.

4. The method according to claim 1, **characterized in that** the reaction temperature of the acylation reaction in step 1 is 80-300 °C, and the reaction time is 3-20 h.

5. The method according to claim 1, **characterized in that** the molar ratio of the catalyst to the compound shown in formula II in step 2 is 0.1:1~20:1.

6. The method according to claim 1, **characterized in that** the solvent for the glycosylation reaction in step 2 is one or more of methanol, ethanol, acetone, butanone, dimethylformamide, dimethylacetamide, dimethyl sulfoxide, tetrahydrofuran and dichloromethane.

7. The method according to claim 1, **characterized in that** the temperature for the glycosylation reaction in step 2 is 0~200 °C, and the reaction time is 3~24 h.

8. The method according to claim 1, **characterized in that** the inorganic acid in step 3 is sulfuric acid or nitric acid.

9. The method according to claim 1, **characterized in that** the solvent for the hydrolysis reaction in step 3 is one or more of acetone, water, methanol, ethanol, dichloromethane, dimethyl sulfoxide, tetrahydrofuran or dioxane.

10. The method according to claim 1, **characterized in that** the volume ratio of the inorganic acid to the solvent for the hydrolysis reaction in step 3 is 0.005:1~0.1:1.

11. The method according to claim 1, **characterized in that** the temperature for the hydrolysis reaction in step 3 is 30 °C~250 °C, and the reaction time is 5~20 h.

12. The method according to claim 1, **characterized in that** the inorganic base in step 4 is one or more of sodium hydroxide, lithium hydroxide, potassium hydroxide, potassium carbonate and sodium carbonate.

13. The method according to claim 1, **characterized in that** the molar ratio of the inorganic base to the compound shown in formula III in step 4 is 0.1:1~50:1.

14. The method according to claim 1, **characterized in that** the temperature of the reaction in step 4 is -10 °C~40 °C, and the reaction time is 1~10 h.

15. The method according to claim 1, **characterized in that** the inorganic acid in step 4 is one or more of hydrochloric acid, sulfuric acid, acetic acid, formic acid, nitric acid or phosphoric acid.

## Patentansprüche

1. Verfahren zur Herstellung von 5,6,4'-Trihydroxyflavon-7-O-D-glucuronsäure, **dadurch gekennzeichnet, dass** das Verfahren umfasst:
Schritt 1: eine Acylierungsreaktion von 5,6,7,4'-Tetrahydroxyflavon mit einem Acylierungsreagens, um eine Verbindung wie in Formel II gezeigt zu erzeugen, wobei das Acylierungsreagens Pivaloylchlorid, Benzoylchlorid, Essigsäureanhydrid, Essigsäure oder Acetylchlorid ist;
Schritt 2: eine Glycolysierungsreaktion einer Acyloxygruppe an Position 7 der Verbindung, wie in Figur II gezeigt, mit Methyl-α-Bromtriacetoxyglucuronat in Gegenwart eines Katalysators, um eine Verbindung wie in Formel III gezeigt zu erzeugen, wobei der Katalysator einer oder mehrere von Kaliumiodid und Kaliumbromid ist;
Schritt 3: eine Hydrolysereaktion der Verbindung wie in Formel III gezeigt unter der Katalyse einer anorganischen Säure, um eine Verbindung wie in Formel IV gezeigt, zu erzeugen; und
Schritt 4: eine Hydrolysereaktion der Verbindung wie in Formel IV gezeigt, unter Katalyse einer anorganischen Base unter einer sauerstofffreien Bedingung, gefolgt durch eine Neutralisierungsreaktion durch Zugabe einer anorganischen Säure, um den pH der Reaktionslösung auf 2~3 einzustellen, um so die 5,6,4'-Trihydroxyflavon-7-O-D-Glucuronsäure zu erhalten; 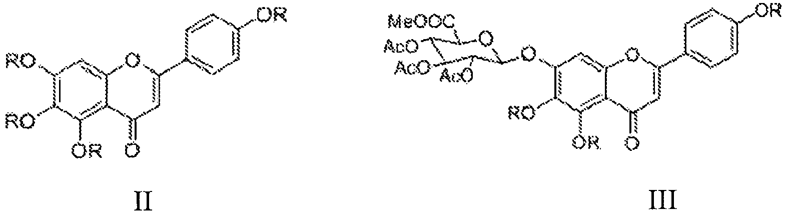 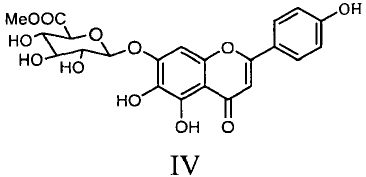 wobei R ausgewählt ist aus der Gruppe bestehend aus Trimethylacetyl, Benzoyl oder Acetyl; Me ist Methyl; und Ac ist Acetyl.

2. Verfahren nach Anspruch 1, **dadurch gekennzeichnet, dass** das molare Verhältnis des Acylierungsreagens zu dem 5,6,7,4'-Tetrahydroxyflavon in Schritt 1 5:1~50:1 ist.

3. Verfahren nach Anspruch 1, **dadurch gekennzeichnet, dass** der Katalysator der Acylierungsreaktion in Schritt 1 einer oder mehrere von Pyridin, Dichlormethan, Toluol, Tetrahydrofuran, 1,4-Dioxan und Ethylacetat ist.

4. Verfahren nach Anspruch 1, **dadurch gekennzeichnet, dass** die Reaktionstemperatur der Acylierungsreaktion in Schritt 1 80~300 °C ist, und die Reaktionszeit 3~20 h ist.

5. Verfahren nach Anspruch 1, **dadurch gekennzeichnet, dass** das molare Verhältnis des Katalysators zu der Verbindung wie in Formel II gezeigt in Schritt 2 0,1:1~20:1 ist.

6. Verfahren nach Anspruch 1, **dadurch gekennzeichnet, dass** das Lösungsmittel für die Glycosylierungsreaktion in Schritt 2 eines oder mehrere von Methanol, Ethanol, Aceton, Butanon, Dimethylformamid, Dimethylacetamid, Dimethylsulfoxid, Tetrahydrofuran und Dichlormethan ist.

7. Verfahren nach Anspruch 1, **dadurch gekennzeichnet, dass** die Temperatur für die Glycosylierungsreaktion in Schritt 2 0-200 °C ist, und die Reaktionszeit 3-24 h ist.

8. Verfahren nach Anspruch 1, **dadurch gekennzeichnet, dass** die anorganische Säure in Schritt 3 Schwefelsäure oder Salpetersäure ist.

9. Verfahren nach Anspruch 1, **dadurch gekennzeichnet, dass** das Lösungsmittel für die Hydrolysereaktion in Schritt 3 eines oder mehrere von Aceton, Wasser, Methanol, Ethanol, Dichlormethan, Dimethylsulfoxid, Tetrahydrofuran oder Dioxan ist.

10. Verfahren nach Anspruch 1, **dadurch gekennzeichnet, dass** das Volumenverhältnis der anorganischen Säure zu dem Lösungsmittel der Hydrolysereaktion in Schritt 3 0,005:1~0,1:1 ist.

11. Verfahren nach Anspruch 1, **dadurch gekennzeichnet, dass** die Temperatur für die Hydrolysereaktion in Schritt 3 30 °C~250 °C ist, und die Reaktionszeit 5~20 h ist.

12. Verfahren nach Anspruch 1, **dadurch gekennzeichnet, dass** die anorganische Base in Schritt 4 eine oder mehrere von Natriumhydroxid, Lithiumhydroxid, Kaliumhydroxid, Kaliumcarbonat und Natriumcarbonat ist.

13. Verfahren nach Anspruch 1, **dadurch gekennzeichnet, dass** das molare Verhältnis der anorganischen Base zu der Verbindung, die in Formel III gezeigt ist, in Schritt 4 0,1:1~50:1 ist.

14. Verfahren nach Anspruch 1, **dadurch gekennzeichnet, dass** die Temperatur der Reaktion in Schritt 4 -10 °C~40 °C ist, und die Reaktionszeit 1~10 h ist.

15. Verfahren nach Anspruch 1, **dadurch gekennzeichnet, dass** die anorganische Säure in Schritt 4 eine oder mehrere von Salzsäure, Schwefelsäure, Essigsäure, Ameisensäure, Salpetersäure oder Phosphorsäure ist.

## Revendications

1. Procédé de préparation d'acide 5,6,4'-trihydroxyflavone-7-O-D-glucuronique, **caractérisé en ce que** le procédé comprend :
étape 1 : une réaction d'acylation de 5,6,7,4'-tétrahydroxyflavone avec un réactif d'acylation, pour produire un composé illustré par la formule II, dans laquelle le réactif d'acylation est le chlorure de pivaloyle, le chlorure de benzoyle, l'anhydride acétique, l'acide acétique ou le chlorure d'acétyle ;
étape 2 : une réaction de glycosylation d'un groupe acyloxy sur la position 7 du composé illustré par la formule II avec de l'α-bromotriacétoxyglucuronate de méthyle en présence d'un catalyseur, pour produire un composé illustré par la formule III, le catalyseur étant un ou plusieurs choisis parmi l'iodure de potassium et le bromure de potassium ;
étape 3 : une réaction d'hydrolyse du composé illustré par la formule III sous catalyse d'un acide inorganique, pour produire un composé illustré par la formule IV ; et
étape 4 : une réaction d'hydrolyse du composé illustré par la formule IV sous catalyse d'une base inorganique dans des conditions sans oxygène, suivie par une réaction de neutralisation par addition d'un acide inorganique pour ajuster le pH de la solution réactionnelle à 2 à environ 3, obtenant ainsi l'acide 5,6,4'-trihydroxyflavone-7-O-D-glucuronique ; 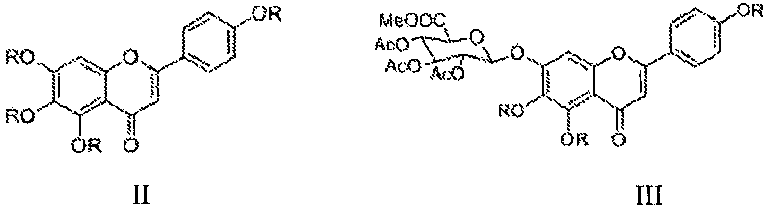 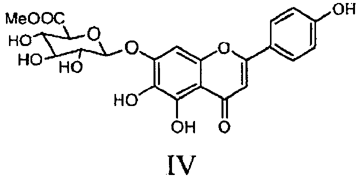 où R est choisi dans le groupe constitué par un groupe triméthylacétyle, benzoyle ou acétyle ; Me est un groupe méthyle ; et Ac est un groupe acétyle.

2. Procédé selon la revendication 1, **caractérisé en ce que** le rapport molaire du réactif d'acylation à la 5,6,7,4'-tétrahydroxyflavone de l'étape 1 est de 5:1 à environ 50:1.

3. Procédé selon la revendication 1, **caractérisé en ce que** le catalyseur pour la réaction d'acylation de l'étape 1 est un ou plusieurs choisis parmi la pyridine, le dichlorométhane, le toluène, le tétrahydrofurane, le 1,4-dioxane et l'acétate d'éthyle.

4. Procédé selon la revendication 1, **caractérisé en ce que** la température réactionnelle de la réaction d'acylation de l'étape 1 est de 80 à environ 300°C, et le temps de réaction est de 3 à environ 20 h.

5. Procédé selon la revendication 1, **caractérisé en ce que** le rapport molaire du catalyseur au composé illustré par la formule II de l'étape 2 est de 0,1:1 à environ 20:1.

6. Procédé selon la revendication 1, **caractérisé en ce que** le solvant pour la réaction de glycosylation de l'étape 2 est un ou plusieurs choisis parmi le méthanol, l'éthanol, l'acétone, la butanone, le diméthylformamide, le diméthylacétamide, le diméthylsulfoxyde, le tétrahydrofurane et le dichlorométhane.

7. Procédé selon la revendication 1, **caractérisé en ce que** la température pour la réaction de glycosylation de l'étape 2 est de 0 à environ 200°C et le temps de réaction est de 3 à environ 24 h.

8. Procédé selon la revendication 1, **caractérisé en ce que** l'acide inorganique de l'étape 3 est l'acide sulfurique ou l'acide nitrique.

9. Procédé selon la revendication 1, **caractérisé en ce que** le solvant pour la réaction d'hydrolyse de l'étape 3 est un ou plusieurs choisis parmi l'acétone, l'eau, le méthanol, l'éthanol, le dichlorométhane, le diméthylsulfoxyde, le tétrahydrofurane ou le dioxane.

10. Procédé selon la revendication 1, **caractérisé en ce que** le rapport en volume de l'acide inorganique au solvant pour la réaction d'hydrolyse de l'étape 3 est de 0,005:1 à environ 0,1:1.

11. Procédé selon la revendication 1, **caractérisé en ce que** la température pour la réaction d'hydrolyse de l'étape 3 est de 30°C à environ 250°C et le temps de réaction est de 5 à environ 20 h.

12. Procédé selon la revendication 1, **caractérisé en ce que** la base inorganique de l'étape 4 est une ou plusieurs choisies parmi l'hydroxyde de sodium, l'hydroxyde de lithium, l'hydroxyde de potassium, le carbonate de potassium et le carbonate de sodium.

13. Procédé selon la revendication 1, **caractérisé en ce que** le rapport molaire de la base inorganique au composé illustré par la formule III de l'étape 4 est de 0,1:1 à environ 50:1.

14. Procédé selon la revendication 1, **caractérisé en ce que** la température de la réaction de l'étape 4 est de -10°C à environ 40°C et le temps de réaction est de 1 à environ 10 h.

15. Procédé selon la revendication 1, **caractérisé en ce que** l'acide inorganique de l'étape 4 est un ou plusieurs choisis parmi l'acide chlorhydrique, l'acide sulfurique, l'acide acétique, l'acide formique, l'acide nitrique ou l'acide phosphorique.
